# EUROPEAN PATENT SPECIFICATION

(11) **EP 3 925 643 B1**
(45) Date of publication and mention of the grant of the patent: **27.11.2024**
(21) Application number: 20770787.8
(22) Date of filing: 25.02.2020
(51) Int. Cl.: A61M 5/142, A61M 5/158, A61M 5/14, A61M 5/162

(54) **LIQUID MEDICINE ADMINISTRATION APPARATUS**
VORRICHTUNG ZUR VERABREICHUNG VON FLÜSSIGEN MEDIKAMENTEN
APPAREIL D'ADMINISTRATION DE MÉDICAMENT LIQUIDE

(30) Priority: 13.03.2019 JP 2019045414
(43) Date of publication of application: 22.12.2021
(73) Proprietor: TERUMO Kabushiki Kaisha, Tokyo 151-0072 (JP)
(72) Inventor: UCHIYAMA, Joji, Ashigarakami-gun, Kanagawa 259-0151 (JP); YAMAZAKI, Tsuyoshi, Ashigarakami-gun, Kanagawa 259-0151 (JP)
(74) Representative: Casalonga
(86) International application number: PCT/JP2020/007327
(87) International publication number: WO 2020/184160

(56) References cited:
- WO-A1-2016/075976
- WO-A1-2018/066378
- JP-A- 2017 136 271
- US-A1- 2008 188 810
- US-A1- 2009 163 874

## Description

### Technical Field

The present invention relates to a drug solution administration apparatus, for example, a drug solution administration apparatus for performing continuous drug solution administration such as an insulin pump. In particular, the present invention relates to a drug solution administration apparatus according to the preamble of independent claim 1.

### Background Art

In recent years, treatments in which drug solution is continuously administered into the body of a patient is performed by subcutaneous injection or intravenous injection. For example, as a treatment for diabetic patients, a continuous injection of a trace amount of insulin into the body of a patient has been implemented. In this treatment, a portable drug solution administration apparatus (so-called insulin pump) that can be fixed to the body or clothing of a patient and carried around is used to administer the drug solution (insulin) to the patient throughout the day.

In such a portable drug solution administration apparatus, the cradle device is first affixed to the skin of a patient. Next, a connection port is mounted on the cradle device, and a cannula provided in the connection port is punctured and indwelt in the patient. The pump main body, which stores and delivers the drug solution, is mounted on the cradle device. At this time, the connecting needle tube that delivers the drug solution from the pump main body is connected to the connection port (for example, refer to PTL 1) .

### Citation List

### Patent Literature

PTL 1: JP-T-2007-509661
PTL 2: WO 2018/066378 A1 relates to an infusion device divided into two portions. The device comprises rails and grooves for connection of the two portions.

### Summary of Invention

### Technical Problem

However, in the technology described in PTL 1, in a case where the cradle device was deformed, there is a concern that the connecting needle tube of the pump main body is punctured diagonally against a plug body provided in the connection port, and the connecting needle tube and the connection port are connected to each other in an incorrect state. Furthermore, even when the connecting needle tube is correctly punctured into the connection port, in a case where the cradle device is bent, there is a concern that the plug body is widened by the connecting needle tube, a gap is generated between the connecting needle tube and the plug body, and the drug solution leaks out through this gap.

In consideration of the above-described problem, the object is to provide a drug solution administration apparatus that can puncture the connecting needle tube provided in the pump main body to a correct position of the connection port. Solution to Problem

In order to solve the above-described problem and achieve the object, the present invention provides a drug solution administration apparatus according to independent claim 1. The dependent claims relate

### Advantageous Effect

According to the drug solution administration apparatus having the above-described configuration, the connecting needle tube provided in the pump main body can be punctured at the correct position of the connection port.

### Brief Description of Drawings

[Fig. 1] Fig. 1 is a perspective view showing a drug solution administration apparatus according to a first embodiment.
[Fig. 2] Fig. 2 is a perspective view showing a state before a pump main body is mounted on a cradle device in the drug solution administration apparatus according to the first embodiment.
[Fig. 3] Fig. 3 is a perspective view showing the pump main body of the drug solution administration apparatus according to the first embodiment.
[Fig. 4] Fig. 4 is a plan view showing an inner configuration of the pump main body of the drug solution administration apparatus according to the first embodiment.
[Fig. 5] Fig. 5 is a cross-sectional view showing a connection state of a connecting needle tube and a connection port of the drug solution administration apparatus according to the first embodiment.
[Fig. 6] Fig. 6 is a plan view showing a positional relationship of the connecting needle tube and a guide groove portion in the pump main body of the drug solution administration apparatus according to the first embodiment.
[Fig. 7] Fig. 7 is a perspective view showing the cradle device and the connection port in the drug solution administration apparatus according to the first embodiment.
[Fig. 8] Fig. 8 is a view showing a state where the pump main body is mounted on the cradle device in the drug solution administration apparatus according to the first embodiment, Fig. 8A is a side view showing a start of engagement of the guide groove portion and a guide rail, and Fig. 8B is a cross-sectional view.
[Fig. 9] Fig. 9 is a view showing a state where the pump main body is mounted on the cradle device in the drug solution administration apparatus according to the first embodiment, Fig. 9A is a side view showing a state where the connecting needle tube is attached to a cap, and Fig. 9B is a cross-sectional view.
[Fig. 10] Fig. 10 is a view showing a state where the pump main body is mounted on the cradle device in the drug solution administration apparatus according to the first embodiment, Fig. 10A is a side view showing a state where the connecting needle tube is punctured to the cap, and Fig. 10B is a cross-sectional view.
[Fig. 11] Fig. 11 is a cross-sectional view showing a drug solution administration apparatus according to a second embodiment.

### Description of Embodiments

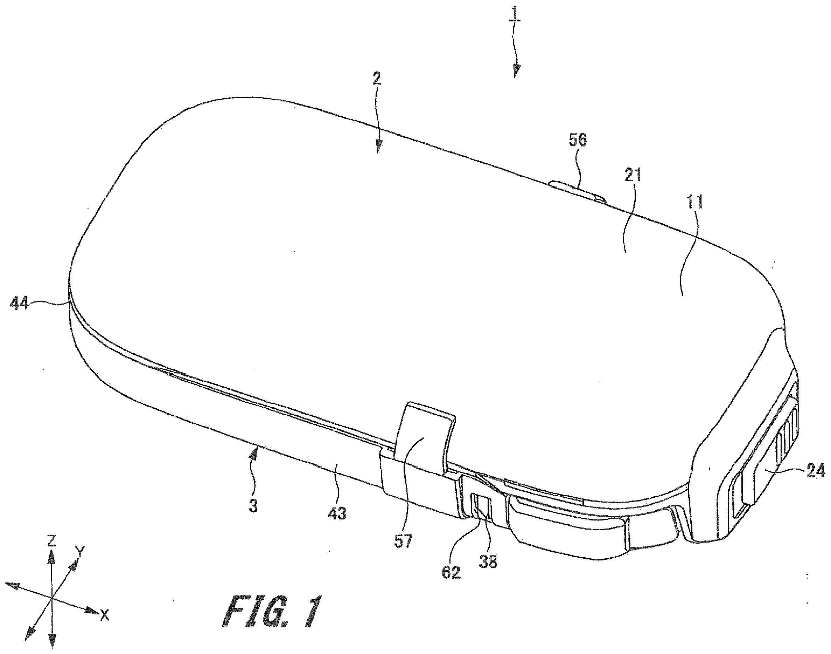

Hereinafter, embodiments of a drug solution administration apparatus will be described with reference to Figs. 1 to 11. Note that the common members in each drawing will be given the same reference numerals. The present invention is not limited to the following aspects.

### 1. First Embodiment

### 1-1. Configuration of Drug Solution Administration Apparatus

First, a configuration example of the drug solution administration apparatus according to a first embodiment (hereinafter referred to as "this example") will be described with reference to Figs. 1 to 5.

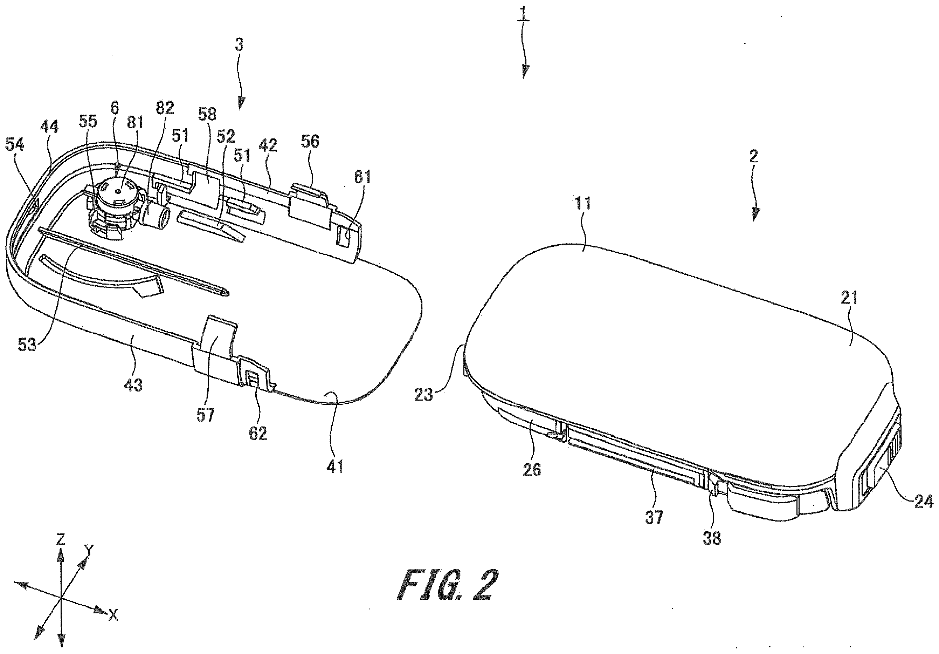

Fig. 1 is a perspective view showing a drug solution administration apparatus, and Fig. 2 is a perspective view showing a state before a pump main body is mounted on a cradle device in the drug solution administration apparatus.

An apparatus shown in Fig. 1 is a portable insulin pump for continuous drug solution administration into the body of a patient, such as a patch type, tube type, or even other portable drug solution administration apparatuses. The drug solution administration apparatus 1 is affixed to a living body to be used. The drug solution administration apparatus 1 includes a pump main body 2, a cradle device 3 on which the pump main body 2 is attachably and detachably mounted, and a connection port 6 mounted on the cradle device 3. The pump main body 2 and the cradle device 3 are assembled by a repeatedly detachable and attachable engagement structure section. As the engagement structure section, various structures can be applied as appropriate.

### [Pump Main Body]

The configuration of the pump main body 2 is described with reference to Figs. 1 to 5.

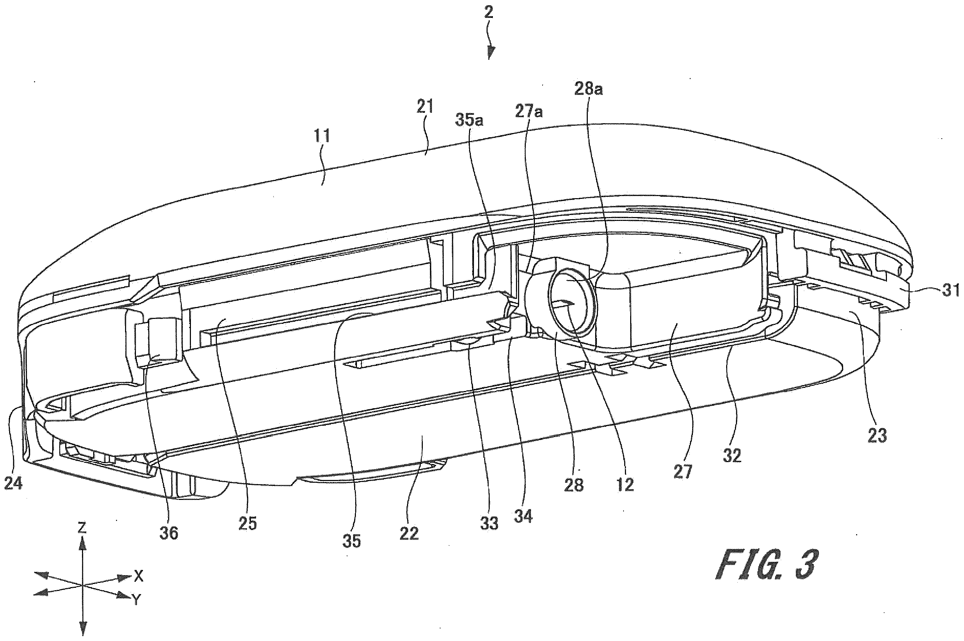

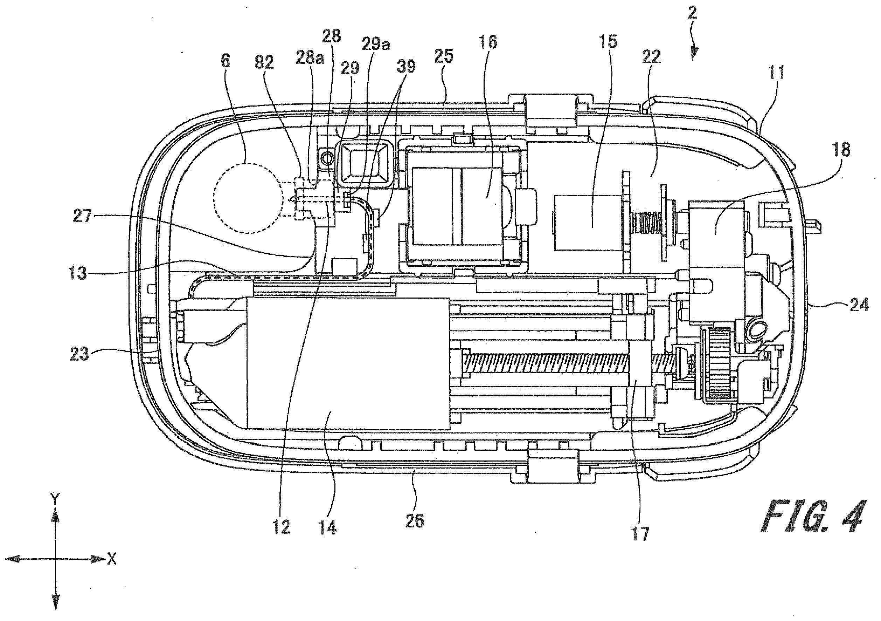

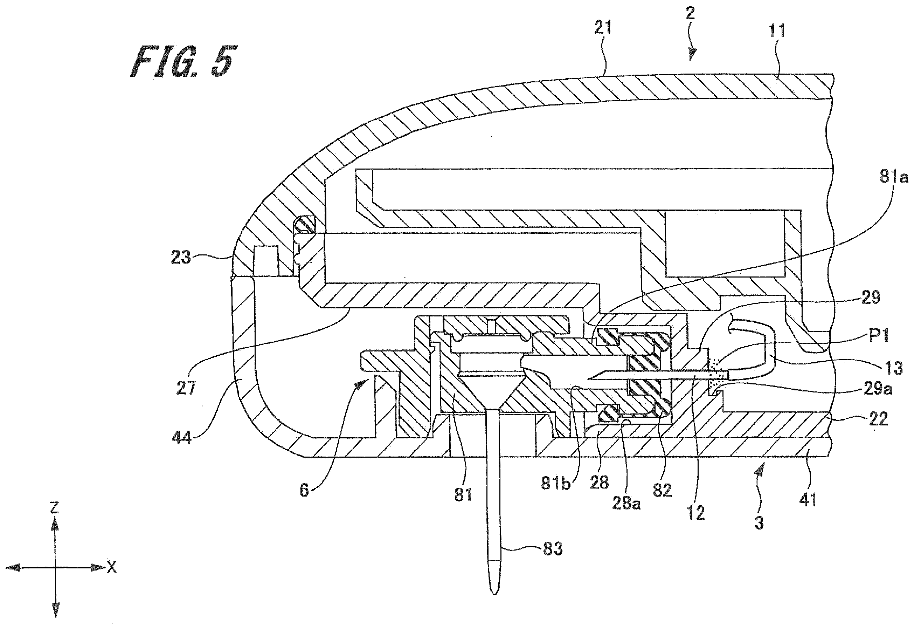

Fig. 3 is a perspective view of the pump main body 2, and Fig. 4 is a plan view showing the inner configuration of the pump main body 2. Fig. 5 is a partial cross-sectional view showing a state where the pump main body 2 is mounted on the cradle device 3.

As shown in Figs. 1 to 3, the pump main body 2 includes a housing 11 and a connecting needle tube 12. As shown in Fig. 4, the housing 11 accommodates a liquid delivery pipe 13, a drug solution storage section 14, a driving section 15, a power supply section 16 that supplies power to the driving section 15, and a plunger mechanism 17 and a transmission mechanism 18 which are actuators. The drug solution storage section 14 is filled with the drug solution to be administered. The drug solution storage section 14 is connected to the liquid delivery pipe 13. On the drug solution storage section 14, the plunger mechanism 17 that pushes out the drug solution is mounted.

The transmission mechanism 18 is connected to the plunger mechanism 17. The transmission mechanism 18 is connected to the driving section 15, and transmits the driving force of the driving section 15 to the plunger mechanism 17 via gears, and the like. As the driving force is transmitted, the plunger mechanism 17 is driven. In this manner, by driving the plunger mechanism 17 and the transmission mechanism 18 which are actuators, the drug solution filled in the drug solution storage section 14 is delivered toward the liquid delivery pipe 13.

The liquid delivery pipe 13 is a pipe disposed between the drug solution storage section 14 and the connecting needle tube 12. One end portion of the liquid delivery pipe 13 is connected to the drug solution storage section 14. The liquid delivery pipe 13 is supported by a positioning section 39 that protrudes from the bottom surface portion 22 of the housing 11, which will be described later. One end portion of the liquid delivery pipe 13 and the other end portion on the opposite side are connected to the connecting needle tube 12 and used as a flow path of the drug solution. The drug solution that has passed through the liquid delivery pipe 13 is discharged from the connecting needle tube 12. Note that the liquid delivery pipe 13 is provided with at least one bent portion formed by bending an appropriate pipe member. In the present embodiment, the liquid delivery pipe 13 has three bent portions.

As shown in Figs. 4 and 5, the connecting needle tube 12 is punctured into a cap 82 provided in a connection section 81a of the connection port 6, which will be described later, when the pump main body 2 is mounted on the cradle device 3. Accordingly, the liquid delivery pipe 13 and the connection port 6 are fluid-connected to each other via the connecting needle tube 12.

Stainless steel is applied as the liquid delivery pipe 13 and the connecting needle tube 12, for example, the austenitic stainless steel prescribed in ISO9626, or the material prescribed in JIS G 4305 cold-rolled stainless steel sheet and strip. In the present embodiment, SUS304 is preferable.

Hereafter, by sliding the pump main body 2 in the direction from the first end to the second end of the cradle device 3, a direction (mounting direction) in which the pump main body 2 is mounted on the cradle device 3 is a first direction X. The direction orthogonal to the first direction X and orthogonal to the direction in which the pump main body 2 and the cradle device 3 overlap each other is a second direction Y (width direction). The second direction Y is substantially parallel to the living body surface when the drug solution administration apparatus 1 administers the drug solution. The direction in which the pump main body 2 and the cradle device 3 overlap each other, that is, the direction orthogonal to both the first direction X and the second direction Y is a third direction Z (up-down direction) . Here, the third direction Z is a direction that is orthogonal to the living body surface when the drug solution administration apparatus 1 administers the drug solution.

As shown in Figs. 2 and 3, the housing 11 is formed in a flat and substantially rectangular parallelepiped shape with curved corner portions. The housing 11 has the upper surface portion 21, a bottom surface portion 22, a front surface portion 23, a back surface portion 24, a first side surface portion 25, a second side surface portion 26, and a bottom surface accommodation section 27. The upper surface portion 21 and the bottom surface portion 22 are facing each other. The upper surface portion 21 is one surface positioned opposite to the side (bottom surface portion 22) where the upper surface portion 21 is mounted on the cradle device 3, in the pump main body 2. The upper surface portion 21 is formed in a substantially rectangular shape with curved corner portions, in the upper view. The upper surface portion 21 has the center portion bulging out in the third direction Z toward the opposite side of the bottom surface portion 22.

The front surface portion 23 substantially perpendicularly communicates with a second end portion of the upper surface portion 21 in the first direction X, and the back surface portion 24 substantially perpendicularly communicates with a first end portion of the upper surface portion 21 in the first direction X. The front surface portion 23 and the back surface portion 24 are facing each other. Furthermore, the first side surface portion 25 substantially perpendicularly communicates with one end portion of the upper surface portion 21 in the second direction Y, and the second side surface portion 26 substantially perpendicularly communicates with the other end portion of the upper surface portion 21 in the second direction Y.

As shown in Fig. 3, an engagement projection 31 is formed in the front surface portion 23. In a case where the front surface portion 23 is viewed from the second end in the first direction X toward the first end, the engagement projection 31 is formed on the other end portion side of the front surface portion 23 in the second direction Y. The engagement projection 31 protrudes from the front surface portion 23 toward the second end portion in the first direction X. The engagement projection 31 is engaged with an engagement hole 54 provided in the cradle device 3 described below.

The bottom surface accommodation section 27 is formed in the corner portion of the bottom surface portion 22, the first side surface portion 25, and the front surface portion 23 in the housing 11. The bottom surface accommodation section 27 is a space which is recessed with a predetermined length (thickness) and a predetermined area (width) from the bottom surface portion 22 to the upper surface portion 21 along the third direction Z.

A tubular connection opening 28 is formed on a wall surface 27a on the first end portion side in the first direction X in the bottom surface accommodation section 27. The connection opening 28 has a tube hole 28a that protrudes from the wall surface 27a toward the second end portion side in the first direction X (that is, in the mounting direction) . The connecting needle tube 12 is disposed in the tube hole 28a of the connection opening 28. The tube hole 28a protects the connecting needle tube 12 by protruding from the wall surface 27a to surround the connecting needle tube 12. The connecting needle tube 12 protrudes from the wall surface 27a in the tube hole 28a toward the second end portion side in the first direction X (in the mounting direction) . In other words, the direction in which the connection opening 28 protrudes from the wall surface 27a is parallel to the first direction X.

As shown in Figs. 4 and 5, when the pump main body 2 is mounted on the cradle device 3, the connection port 6 attached to the cradle device 3 is accommodated in the bottom surface accommodation section 27. Furthermore, the connection section 81a and the cap 82 of the connection port 6 are inserted into the tube hole 28a of the connection opening 28. The connecting needle tube 12 protrudes from the wall surface 27a in the tube hole 28a toward the second end portion side in the first direction X (that is, in the mounting direction) . In other words, the direction in which the connecting needle tube 12 protrudes from the wall surface 27a is parallel to the first direction X.

A needle support section 29 is formed on the inside of the housing 11 at the connection opening 28. The needle support section 29 supports the connecting needle tube 12. A concave portion 29a is formed in the needle support section 29. The needle support section 29 supports the connecting needle tube 12 from the second direction Y and the third direction Z. The concave portion 29a is filled with an adhesive P1 (refer to Fig. 5) for fixing the connecting needle tube 12. Accordingly, the connecting needle tube 12 can be reliably fixed to the needle support section 29. Accordingly, it is possible to prevent the positional deviation of the connecting needle tube 12 caused by the operation of connecting the pump main body 2 to the cradle device 3.

As shown in Fig. 3, the bottom surface portion 22 has a sliding groove portion 32 and a detection groove portion 34. The sliding groove portion 32 and the detection groove portion 34 are concave portions recessed from the bottom surface portion 22 toward the upper surface portion 21 along the third direction Z. The sliding groove portion 32 is formed in the middle of the second direction Y in the bottom surface portion 22. The sliding groove portion 32 is formed continuously from the second end portion to the first end portion in the first direction X in the bottom surface portion 22.

The detection groove portion 34 is formed on one end portion side in the second direction Y in the bottom surface portion 22. The detection groove portion 34 extends from the wall surface 27a of the bottom surface accommodation section 27 across the first end portion in the first direction X. In the detection groove portion 34, a mounting detection switch 33 is provided.

The mounting detection switch 33 is pressed by a detection rail 52 provided in the cradle device 3, which will be described later, when the pump main body 2 is mounted on the cradle device 3. Then, when the mounting detection switch 33 is pressed, the mounting information to the control unit, which is not shown, is output. Accordingly, the control unit detects that the pump main body 2 has been mounted on the cradle device 3.

As shown in Fig. 4, the positioning section 39 is provided inside the bottom surface portion 22. The positioning section 39 is a projection that protrudes substantially perpendicularly from the inner surface in the bottom surface portion 22. The positioning section 39 is disposed in the vicinity of the needle support section 29. The positioning section 39 supports the liquid delivery pipe 13 and positions the liquid delivery pipe 13.

As shown in Fig. 3, in the present embodiment, the pump main body 2 and the cradle device 3 have a repeatedly detachable and attachable engagement structure section. The engagement structure section, for example, has a hook mechanism. Specifically, the first side surface portion 25 has the first guide groove portion 35 and the first engagement hook portion 36 as the engagement structure section. The first engagement hook portion 36 is formed at the first end portion of the first side surface portion 25 in the first direction X. The first engagement hook portion 36 is formed on the first end portion side of the first guide groove portion 35 in the first direction X. The first engagement hook portion 36 is attachably and detachably engaged with a first engagement receiving section 61 (refer to Fig. 2) of the cradle device 3, which will be described later.

The first guide groove portion 35 is formed in the middle of the third direction Z in the first side surface portion 25. The first guide groove portion 35 is a groove portion recessed from the first side surface portion 25 into the housing 11 along the second direction Y. The first guide groove portion 35 extends toward the second end portion side along the first direction X from the vicinity of the first engagement hook portion 36 in the first side surface portion 25.

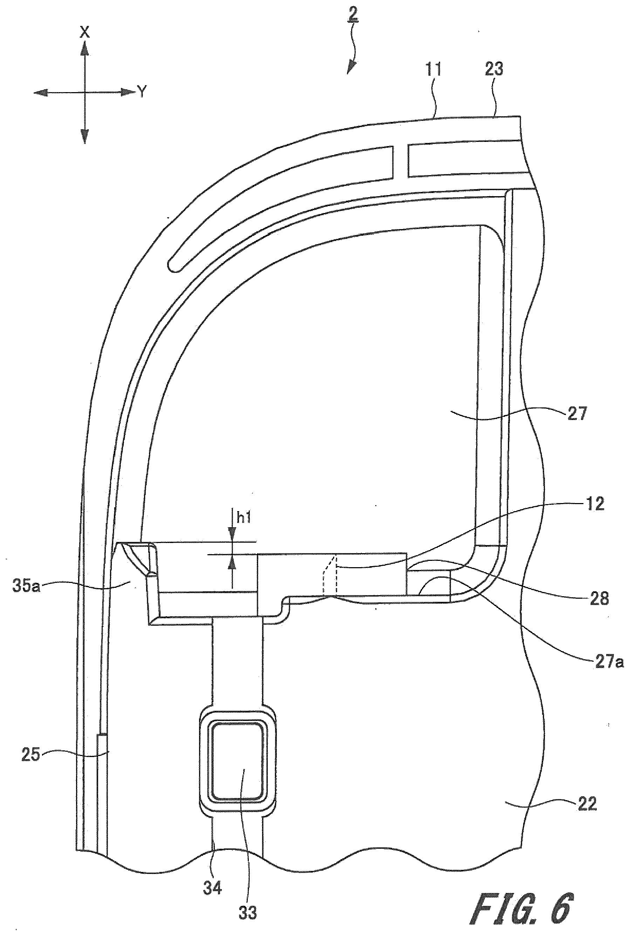

Fig. 6 is a plan view showing the positional relationship of the connecting needle tube 12 and the first guide groove portion 35.

As shown in Figs. 3 to 6, an extension section 35a is provided at the second end portion of the first side surface portion 25, that is, on the bottom surface accommodation section 27 side. As shown in Fig. 6, the extension section 35a is a plate-like portion that protrudes from the wall surface 27a of the bottom surface accommodation section 27 toward the second end portion in the first direction X at the second end portion of the first side surface portion 25. The extension section 35a protrudes toward the second end portion in the first direction X by a length h1 from the connection opening 28 that protrudes from the wall surface 27a. In other words, a protrusion length of the extension section 35a from the wall surface 27a is longer than a protrusion length of the connection opening 28 from the wall surface 27a. The length of the guide groove portion 35 extending from the wall surface of the housing 11 is longer than the length from the wall surface of the housing 11 to the opening surface of the connection opening 28.

The length at which the guide groove portion 35 protrudes from the wall surface of housing 11 is longer than the length at which the connecting needle tube 12 protrudes from the wall surface of the housing 11. More specifically, the extension section 35a extends toward the second end portion side in the first direction X from the distal end of the connecting needle tube 12. Note that the extension section 35a may be formed flush with the first side surface portion 25. Note that it is preferable that the second end portion of the first side surface portion 25 is recessed into the housing 11 along the second direction Y with respect to the surface of the first side surface portion 25. More preferably, the extension section 35a is formed with the same surface as the bottom surface of the recess of the first guide groove portion 35. Accordingly, it is possible to not only prevent sinkage during molding, but also maintain the posture of the connecting needle tube 12 such that the connecting needle tube 12 can be correctly punctured into the cap 82 immediately before and throughout the puncture by positioning the connecting needle tube 12 and the cap 82 closer to each other, as described below.

The first guide groove portion 35 is formed from the vicinity of the first engagement hook portion 36 across the extension section 35a. Therefore, the second end portion of the first guide groove portion 35 in the first direction X is disposed on the second end portion side in the first direction X from the distal end of the connecting needle tube 12 and the connection port 6. By providing the first engagement hook portion 36 and the first engagement receiving section 61 on the first end portion side in the first direction X in the pump main body 2 and the cradle device 3, the length at which the first guide groove portion 35 guides the pump main body 2 can be ensured when attaching and detaching the pump main body 2 to and from the cradle device 3. Accordingly, the guiding function of the first guide groove portion 35 can be strengthened.

It is preferable that a plurality of engagement structure sections are provided. In addition to the first side surface portion 25, it is preferable to provide an engagement structure section on the facing second side surface portion 26. As shown in Fig. 2, the second side surface portion 26 has a second guide groove portion 37 and a second engagement hook portion 38 as the engagement structure section. The second engagement hook portion 38 is formed at the first end portion of the first direction X in the second side surface portion 26. The second engagement hook portion 38 is attachably and detachably engaged with a second engagement receiving section 62 of the cradle device 3, which will be described later. The plurality of engagement structure sections (the first engagement hook portion 36 and the first engagement receiving section 61, the second engagement hook portion 38 and the second engagement receiving section 62) are symmetrically disposed with respect to a central axis of the first direction X in the pump main body 2 and the cradle device 3.

The second guide groove portion 37 is formed in the middle of the third direction Z in the second side surface portion 26. The second guide groove portion 37 is a groove portion recessed from the second side surface portion 26 into the housing 11 along the second direction Y. The second guide groove portion 37 extends from the vicinity of the second engagement hook portion 38 along the first direction X with a length that is substantially the same as the length in the first direction X in the first guide groove portion 35. By providing the second engagement hook portion 38 and the second engagement receiving section 62 on the first end portion side in the first direction X in the pump main body 2 and the cradle device 3, the length at which the second guide groove portion 36 guides the pump main body 2 can be ensured when attaching and detaching the pump main body 2 to and from the cradle device 3. Accordingly, the guiding function of the second guide groove portion 36 can be strengthened.

### [Cradle Device]

Next, the cradle device 3 will be described with reference to Figs. 1, 2, and 7.

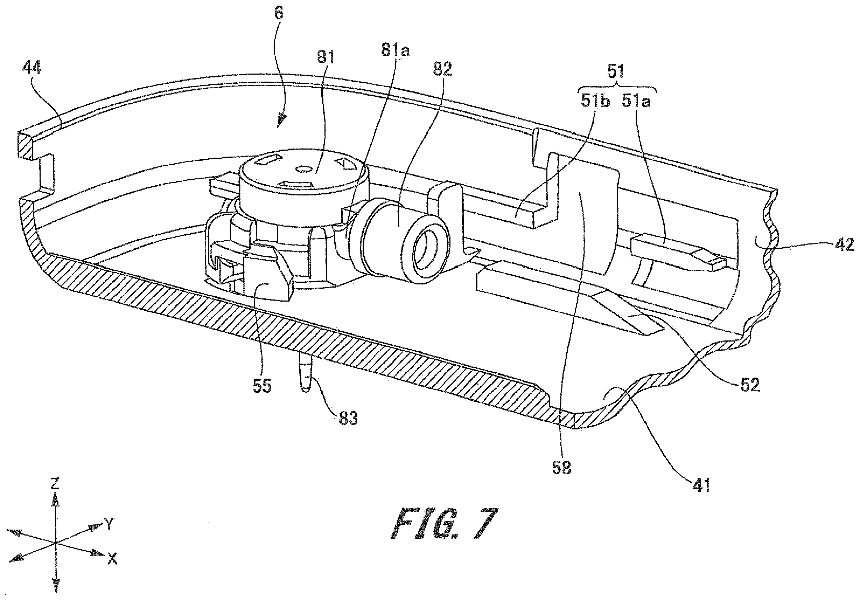

Fig. 7 shows a perspective view in which the cradle device 3 and the connection port 6 are enlarged and showed.

As shown in Fig. 1, the cradle device 3 is configured to be capable of carrying the pump main body 2 having the above-described configuration. As shown in Fig. 2, the cradle device 3 has a substantially flat plate-shaped placement surface portion 41, a first side wall portion 42, a second side wall portion 43, and a third side wall portion 44. The placement surface portion 41 is formed in a substantially rectangular shape with curved corner portions, in the upper view. When the pump main body 2 is mounted on the cradle device 3, the bottom surface portion 22 of the housing 11 of the pump main body 2 is placed on the placement surface portion 41.

On one surface of the placement surface portion 41, the detection rail 52, a sliding rail 53, and a mounting section 55 are provided. The mounting section 55 is formed at the second end portion of the placement surface portion 41 in the first direction X and is formed at one end portion in the second direction Y. As shown in Fig. 6, the connection port 6 is mounted on the mounting section 55. Furthermore, in the mounting section 55, an insertion hole (not shown) through which a cannula 83 of the connection port 6 (will be described later) is inserted is provided.

The detection rail 52 is formed at one end portion of the placement surface portion 41 in the second direction Y, that is, on the first end portion side of the mounting section 55 in the first direction X. The detection rail 52 is a projection portion that protrudes from one surface of the placement surface portion 41. The first end portion of the detection rail 52 has a gradual increase in thickness from the placement surface portion 41 as moving toward the second end portion side in the first direction X. The detection rail 52 extends for a predetermined length along the first direction X. When the pump main body 2 is mounted on the cradle device 3, the detection rail 52 enters the detection groove portion 34 provided on the pump main body 2. The detection rail 52 then presses the mounting detection switch 33.

The sliding rail 53 is formed in the middle of the second direction Y on one surface of the placement surface portion 41. The sliding rail 53 extends from the second end portion in the first direction X on one surface of the placement surface portion 41 toward the first end portion. When the pump main body 2 is mounted on the cradle device 3, the sliding rail 53 is slidably fitted with the sliding groove portion 32 provided in the bottom surface portion 22 of the pump main body 2.

At one end portion of the placement surface portion 41 in the second direction Y, the first side wall portion 42 is continuous substantially perpendicularly to the placement surface portion 41. At the other end portion of the placement surface portion 41 in the second direction Y, the second side wall portion 43 is continuous substantially perpendicularly to the placement surface portion 41. At the second end portion of the placement surface portion 41 in the first direction X, the third side wall portion 44 is continuous substantially perpendicularly to the placement surface portion 41. The first side wall portion 42 and the second side wall portion 43 face each other. Three of the four sides of the placement surface portion 41 is connected to the first side wall portion 42, the second side wall portion 43 and the third side wall portion 44. The first end of the placement surface portion 41 in the first direction X does not have a side wall portion that is continuous and substantially perpendicular to the placement surface portion 41. In other words, the first end of the placement surface portion 41 in the first direction X and the first end portion are open. Accordingly, the pump main body 2 is inserted into the placement surface portion 41 from the first end side in the first direction X.

In a state where the pump main body 2 is mounted on the cradle device 3, the first side wall portion 42 faces the first side surface portion 25 of the housing 11 in the pump main body 2, and the second side wall portion 43 faces the second side surface portion 26. The third side wall portion 44 faces the front surface portion 23 of the housing 11.

As shown in Fig. 2, in the third side wall portion 44, the engagement hole 54 is open. When the pump main body 2 is mounted on the cradle device 3, the engagement hole 54 is engaged with the engagement projection 31 of the pump main body 2.

In the first side wall portion 42, a first guide rail 51, a first posture correction section 56, and the first engagement receiving section 61 are formed. The first engagement receiving section 61 is formed at the first end portion of the first side wall portion 42 in the first direction X. The first engagement receiving section 61 is an opening portion with the first side wall portion 42 cut out in a substantially rectangular shape. When the pump main body 2 is mounted on the cradle device 3, the first engagement hook portion 36 is attachably and detachably engaged with the first engagement receiving section 61.

As shown in Fig. 7, the first guide rail 51 protrudes from one surface facing the second side wall portion 43 in the first side wall portion 42 toward the other end portion in the second direction Y. The first guide rail 51 is a projection portion formed parallel to the first direction X from the second end portion of the first side wall portion 42 in the first direction X. The first guide rail 51 may not necessarily the projection portion which continuously extends. As shown in Figs. 2 and 7, a notch 58 may be provided in the middle of the guide rail 51 as appropriate. In the present embodiment, the first guide rail 51 is formed by a first end portion side first guide rail 51a and a second end portion side first guide rail 51b.

The second end portion of the first guide rail 51 in the first direction X extends to the position of the connection port 6 (which will be described later) attached to the mounting section 55. The first end portion of the first guide rail 51 in the first direction X is positioned on the first end portion side in the first direction X from the connection section 81a and the cap 82 of the connection port 6, which will be described later.

When the pump main body 2 is mounted on the cradle device 3, the first guide rail 51 is engaged with the first guide groove portion 35 provided on the first side surface portion 25 of the pump main body 2. Accordingly, the pump main body 2 is guided in the mounting direction.

As shown in Figs. 1 and 2, the first posture correction section 56 is a plate-like protruding portion extending from the first side wall portion 42. The first posture correction section 56 extends further upward in the third direction Z from the first side wall portion 42. The first posture correction section 56 is formed on the first end portion side of the first guide rail 51 in the first direction X, in the first side wall portion 42.

The first posture correction section 56 protrudes in the third direction Z from one end portion of the first side wall portion 42 in the third direction Z and is curved toward the other end portion in the second direction Y. In other words, the first posture correction section 56 is formed in a shape corresponding to the shape of the connection section (corner portion) between the upper surface portion 21 and the first side surface portion 25 in the housing 11. The distal end and/or the distal portion of the first posture correction section 56 is positioned above the placement surface portion 41 in the third direction Z in a side view from the first direction X of the cradle device 3.

As shown in Figs. 1 and 2, the second side wall portion 43 has a second guide rail (not shown), a second posture correction section 57, and the second engagement receiving section 62. The second engagement receiving section 62 is formed at the first end portion of the second side wall portion 43 in the first direction X. The second engagement receiving section 62 is an opening portion with the second side wall portion 43 cut out in a substantially rectangular shape. When the pump main body 2 is mounted on the cradle device 3, the second engagement hook portion 38 is attachably and detachably engaged with the second engagement receiving section 62.

The second guide rail protrudes from one surface facing the first side wall portion 42 in the second side wall portion 43 toward one end portion in the second direction Y. The second guide rail is a projection portion formed parallel to the direction toward the first end from the second end portion of the second side wall portion 43 in the first direction X. When the pump main body 2 is mounted on the cradle device 3, the second guide rail is engaged with the second guide groove portion 37 provided on the second side surface portion 26 of the pump main body 2. Accordingly, the pump main body 2 is guided in the mounting direction.

The second posture correction section 57 is a plate-like protruding portion extending from the second side wall portion 43. The second posture correction section 57 extends further upward in the third direction Z from the second side wall portion 43. The second posture correction section 57 is formed on the first end portion side of the second guide rail in the first direction X, in the second side wall portion 43. The second posture correction section 57 is formed at a position facing the first posture correction section 56 formed in the first side wall portion 42.

The second posture correction section 57 protrudes in the third direction Z from one end portion of the second side wall portion 43 in the third direction Z and is curved toward one end portion in the second direction Y. In other words, the second posture correction section 57 is formed in a shape corresponding to the shape of the corner portion between the upper surface portion 21 and the second side surface portion 26 in the housing 11. The distal end and/or the distal portion of the second posture correction section 57 is positioned above the placement surface portion 41 in the third direction Z in a side view from the first direction X of the cradle device 3. When the pump main body 2 is mounted on the cradle device 3, the first posture correction section 56 and the second posture correction section 57 correct the posture of the pump main body 2 when the pump main body 2 is mounted.

In the cradle device 3, an affixing sheet that is affixed to the skin of the patient is provided. The affixing sheet is attached to the other surface opposite to one surface of the placement surface portion 41 of the cradle device 3. The affixing sheet has an opening portion (not shown) through which the cannula 83 of the connection port 6 (which will be described below) penetrates.

The affixing sheet is made of a flexible member, and an adhesive layer affixed to the skin of the patient is formed on the surface opposite to the placement surface portion 41. In a state before being affixed to the skin of the patient, the adhesive layer of the affixing sheet is covered with a separation paper.

### [Connection Port]

Next, the connection port 6 will be described with reference to Figs. 5 and 7.

As shown in Figs. 5 and 7, the connection port 6 has a port main body 81 and the cannula 83. The cannula 83 is held in the port main body 81. The port main body 81 has the tubular connection section 81a. The inside of the connection section 81a (tube hole 81b), the port main body 81, and the cannula 83 communicate with each other. The cap 82 is mounted on the distal portion of the connection section 81a, and the other end portion of the connection section 81a is connected to the port main body 81. The cap 82 seals the distal end opening of the connection section 81a. Accordingly, the inside of the connection port 6 is isolated from the external environment.

When the connection port 6 is mounted on the mounting section 55 of the cradle device 3, the cannula 83 penetrates the placement surface portion 41 together with a puncture needle along the third direction Z and protrudes to the other surface (a surface affixed to the skin) of the placement surface portion 41. The cannula 83 is then punctured into the living body together with the puncture needle. After this, by removing the puncture needle, the cannula 83 is indwelt in the living body.

The connection section 81a of the port main body 81 faces the first end portion side of the first direction X, that is, the upstream side of the mounting direction. The connecting needle tube 12 of the pump main body 2 enters the tube hole 81b by puncturing the septum surface of the cap 82. Accordingly, the port main body 81 is connected to the liquid delivery pipe 13 of the pump main body 2, and the liquid delivery pipe 13 is fluid-connected to the cannula 83. The drug solution stored in the drug solution storage section 14 of the pump main body 2 is delivered to the connection port 6 via the liquid delivery pipe 13 and administered to the patient through the cannula 83, by driving the driving section 15.

### 1-2. Mounting Operation Example of Pump Main Body

Next, an example of the mounting operation of mounting the pump main body 2 on the cradle device 3 in the drug solution administration apparatus 1 having the above-described configuration will be described with reference to Figs. 1, 2, and 8 to 10.

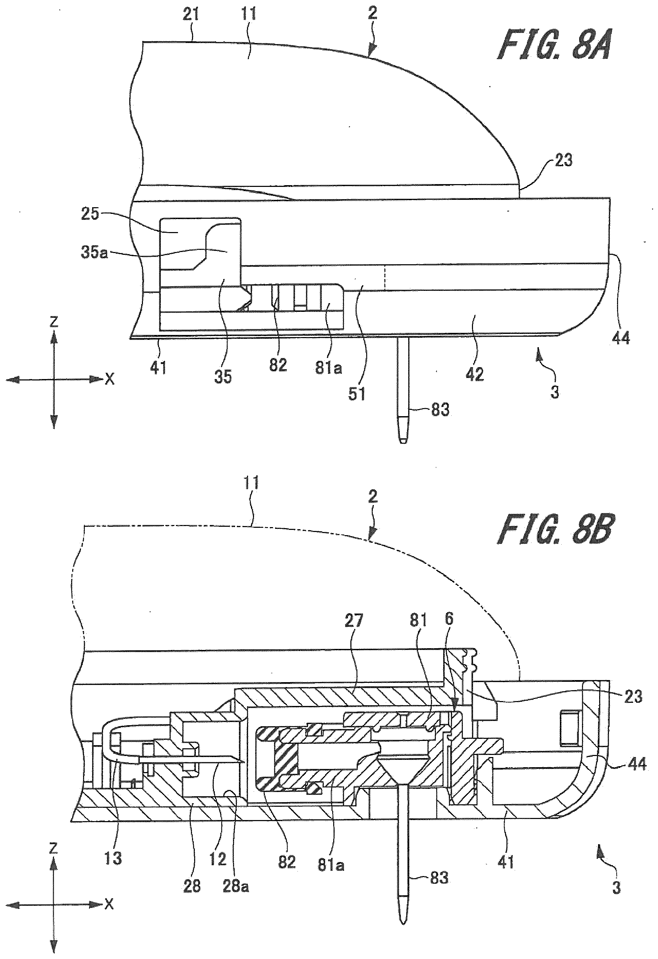

Figs. 8 to 10 are views showing a state where the pump main body 2 is mounted on the cradle device 3.

First, the separation paper of the affixing sheet in the cradle device 3 is peeled off, and the affixing sheet is affixed to the skin of the patient. Accordingly, the cradle device 3 is attached to the skin of the patient via the affixing sheet. Then, as shown in Fig. 2, the connection port 6 is mounted on the mounting section 55 of the cradle device 3, and the cannula 83 is punctured and indwelt in the patient by using a puncture apparatus (not shown) . After the puncture, the puncture apparatus is removed from the cradle device 3 and disposed of.

Next, as shown in Fig. 2, the pump main body 2 is inserted from the first end portion side of the cradle device 3 in the first direction X. Then, the pump main body 2 is made slide along the placement surface portion 41 of the cradle device 3 toward the second end in the first direction X. At this time, the posture of the pump main body 2 at the start of mounting is corrected by attaching the first posture correction section 56 and the second posture correction section 57.

As shown in Figs. 8A and 8B, before the cap 82 of the connection port 6 is inserted into the tube hole 28a of the connection opening 28, a state where the first guide rail 51 is inserted into the first guide groove portion 35 formed in the extension section 35a is maintained. Accordingly, before the pump main body 2 is connected to the cradle device 3, the tilt in the mounting direction of the pump main body 2 on the cradle device 3 is corrected. As a result, the cap 82 and/or the connection section 81a can be inserted straight into the connection opening 28 along the first direction X.

In this example, in the middle of the first guide rail 51, in a state before puncturing the cannula 83, the notch 58 for engaging the puncture apparatus (not shown) is provided. Accordingly, the second end portion side first guide rail 51b and the first end portion side first guide rail 51a are formed across the notch 58 as the first guide rail 51. The second end portion of the extension section 35a goes over the notch 58 before the cap 82 is inserted into the tube hole 82a. More specifically, before the cap 82 is inserted into the tube hole 28a, the first guide groove portion 35 is inserted into the second end portion side first guide rail 51b.

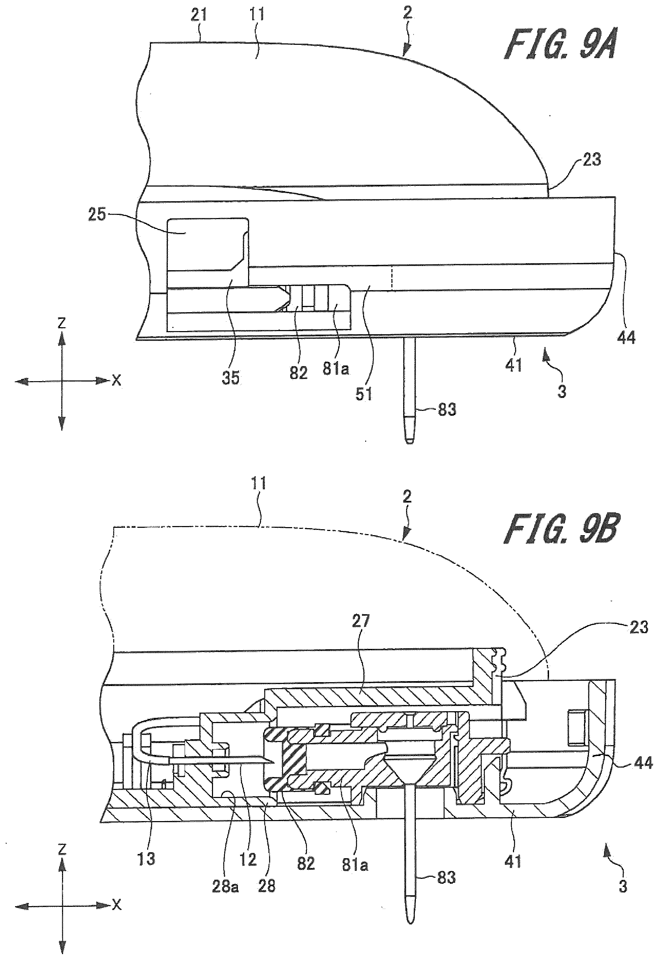

Next, when the pump main body 2 is slid further toward the second end side in the first direction X against the cradle device 3, as shown in Figs. 9A and 9B, the distal end of the connecting needle tube 12 is attached to the cap 82 of the connection port 6. During this time, the first guide groove portion 35 in the pump main body 2 and the first guide rail 51 of the cradle device 3 are maintained to be engaged. Therefore, the pump main body 2 is guided by the first guide groove portion 35 and the first guide rail 51 to be mounted in the correct direction. By providing the extension section 35a in the correct direction, the cap 82 can be inserted into the connection opening 28 and the connecting needle tube 12 can be attached to the septum surface of the cap 82 substantially perpendicularly. Furthermore, the distal end of the connecting needle tube 12 can puncture the septum surface of the cap 82 and keep the puncture direction substantially constant while passing therethrough.

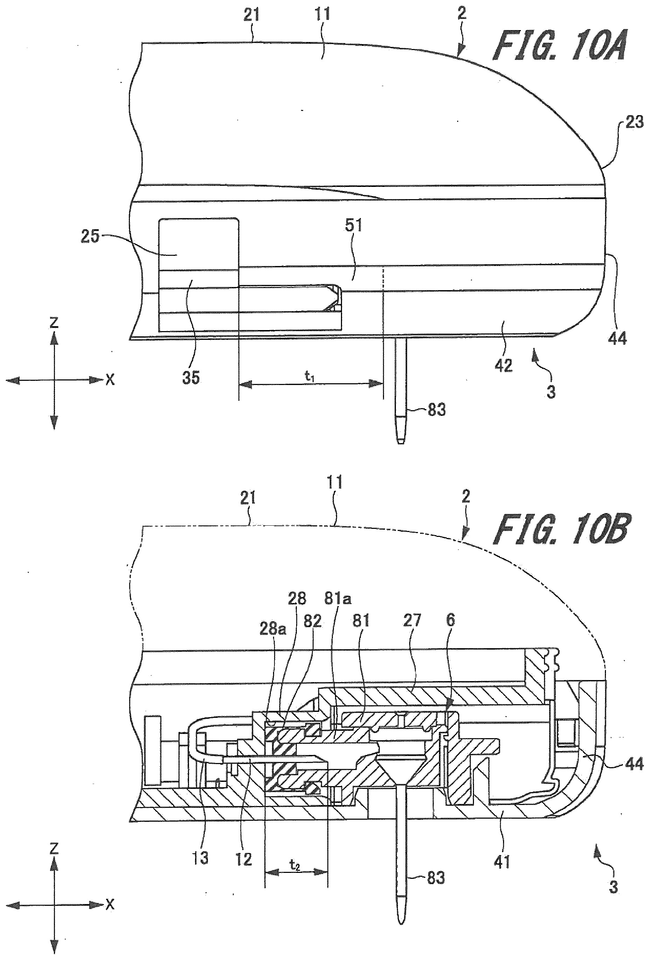

By further sliding the pump main body 2 to the second end in the first direction X, as shown in Figs. 10a and 10b, the cap 82 and the connection section 81a of the connection port 6 are inserted into the tube hole 28a of the connection opening 28. The connection opening 28 holds the cap 82 and the connection section 81a in the tube hole 28a. Accordingly, even when stress is applied to the cradle device 3, it is possible to prevent the cap 82 or the connection section 81a from being deformed.

Then, the connecting needle tube 12 of the pump main body 2 is punctured into the cap 82 of the connection port 6, and the connection port 6 and the liquid delivery pipe 13 communicate with each other. Accordingly, the drug solution storage section 14 of the pump main body 2 and the cannula 83 of the connection port 6 are connected to each other via the liquid delivery pipe 13, the connecting needle tube 12 and the port main body 81.

The engagement projection 31 is engaged with the engagement hole 54 of the cradle device 3. Then, the first engagement hook portion 36 is engaged with the first engagement receiving section 61, and the second engagement hook portion 38 is engaged with the second engagement receiving section 62. Accordingly, the pump main body 2 mounted on the cradle device 3 can be prevented from falling out of the cradle device 3.

The detection rail 52 of the cradle device 3 slides through the detection groove portion 34 of the pump main body 2 and presses the mounting detection switch 33. Accordingly, the control unit (not shown) provided in the pump main body 2 detects that the pump main body 2 has been mounted on the cradle device 3. As a result, the mounting operation of the pump main body 2 onto the cradle device 3 is completed.

According to the drug solution administration apparatus 1 of this example, as the first guide rail 51 is engaged with the first guide groove portion 35, the mounting direction of the pump main body 2 onto the cradle device 3 is guided in the correct direction. Therefore, it is possible to prevent the mounting detection switch 33 from being pressed by the detection rail 52 in an inappropriate mounting state where the pump main body 2 is tilted against the cradle device 3, and to prevent false detection from occurring. As a result, the mounting state of the pump main body 2 to the cradle device 3 is normally detected by the mounting detection switch 33.

As shown in Figs. 10A and 10B, a length t1 at which the first guide rail 51 and the first guide groove portion 35 are engaged with each other is set to be longer than a length t2 at which the connecting needle tube 12 enters the connection port 6 (t1 > t2). Therefore, the strength around the connection port 6 in the cradle device 3 can be increased by the engagement of the first guide rail 51 and the first guide groove portion 35.

In other words, even in a case where stress is applied to the cradle device 3 and the cradle device 3 is bent during the operation of attaching the pump main body 2 to the cradle device 3, the puncture guide function can be enhanced in the vicinity of the connecting needle tube 12 and the cap 82 during the puncture operation from before the connecting needle tube 12 is punctured into the cap 82.

By providing the extension section 35a to one end portion of the first guide groove portion 35, even when the drug solution administration apparatus 1 is in use, it is possible to enhance posture holding force of the connection opening 28, the connection section 81a, and the connecting needle tube 12 around the connection port 6 in the cradle device 3 . Depending on the movement of the living body surface or the external force, there is a case where a difference occurs in the followability to the living body surface between the cradle device 3 and the pump main body 2. At this time, as a part of the pump main body 2 is separated from the cradle device 3, there is a concern that the connecting needle tube 12 widens the cap 82 and the drug solution leaks out. In other words, even in a case where stress is applied to the cradle device 3 and deformation occurs in the placement surface portion 41 and the like, the first guide rail 51 and the first guide groove portion 35 can correct the posture of the connection port 6 and the connecting needle tube 12 of the cradle device 3 so as to be parallel to the first direction X.

Furthermore, by providing the extension section 35a, it is possible to correctly hold the positions of the connection opening 28 and the connecting needle tube 12 in a state of being punctured in the cap 82, and thus, it is possible to prevent the connecting needle tube 12 from widening the puncture portion in the septum surface of the cap 82, or the connecting needle tube 12 or the connection port 6 from deforming. Accordingly, a gap is generated between the connecting needle tube 12 and the cap 82, the drug solution is prevented from leaking out.

Note that, in the above-described embodiment, an example was described in which the projecting first guide rail 51 and the second guide rail are provided on the cradle device 3 side and the first guide groove portion 35 and the second guide groove portion 37 which are engaged with the first guide rail 51 and the second guide rail are provided on the pump main body 2 side, but the present invention is not limited thereto. For example, a guide groove portion may be provided on the cradle device 3 side, and a guide rail which is engaged with the guide groove portion may be provided on the pump main body 2 side.

### 2. Second Embodiment

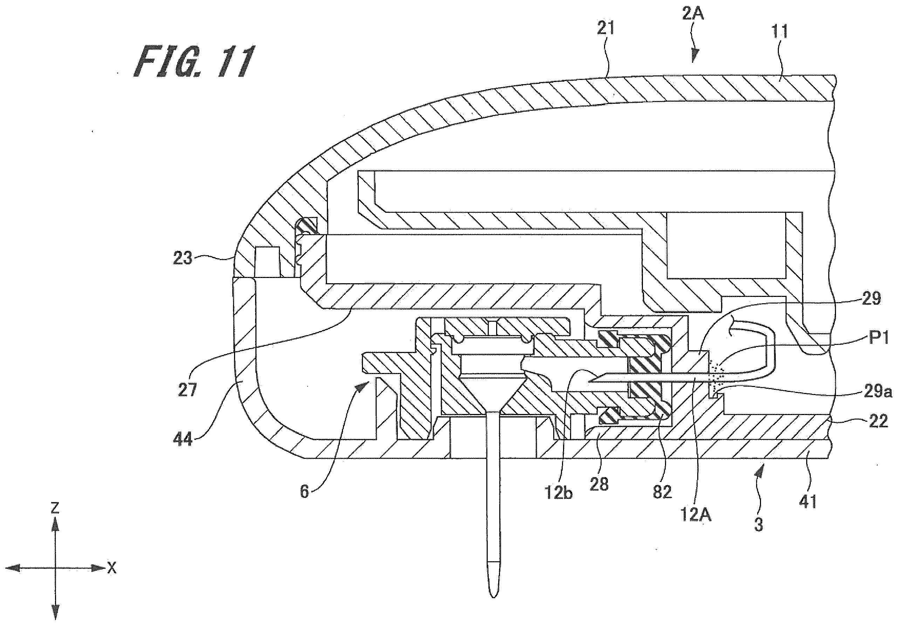

Next, the drug solution administration apparatus according to a second embodiment will be described with reference to Fig. 11.

Fig. 11 is a cross-sectional view showing the drug solution administration apparatus according to the second embodiment. Parts common to the drug solution administration apparatus 1 according to the first embodiment will be given the same reference numerals, and the duplicated description thereof will be omitted.

As shown in Fig. 11, a pump main body 2A of the drug solution administration apparatus according to the second embodiment has a liquid delivery needle tube 12A in which the connecting needle tube and the liquid delivery pipe are integrally formed. In other words, the drug solution storage section 14 and the connection port 6 are connected to each other via a liquid delivery needle tube 12A, which has a needle section at one end portion. Similar to the first embodiment, the liquid delivery needle tube 12A has at least one bent portion. One end portion of the liquid delivery needle tube 12A is connected to the drug solution storage section 14 (refer to Fig. 4). The liquid delivery needle tube 12A is supported by the positioning section 39 for positioning.

The other end portion of the liquid delivery needle tube 12A is supported by the needle support section 29, and the distal portion protrudes outward from the connection opening 28. Then, the other end portion of the liquid delivery needle tube 12A is fixed to the needle support section 29 by the adhesive P1 filled in the concave portion 29a of the needle support section 29. At the distal end of the liquid delivery needle tube 12A, a needle tip 12b that can puncture the cap 82 is formed. Accordingly, the connecting needle tube 12A can be reliably fixed to the needle support section 29. Accordingly, it is possible to prevent the positional deviation of the connecting needle tube 12A caused by the operation of puncturing the pump main body 2 to the cradle device 3. Since fixtures such as needle hub members can be omitted, weight reduction can be achieved.

Stainless steel is applied as the liquid delivery needle tube 12A, for example, the austenitic stainless steel prescribed in ISO9626, or the material prescribed in JIS G 4305 cold-rolled stainless steel sheet and strip. In the present embodiment, SUS304 is preferable.

The other configurations are the same as those of the drug solution administration apparatus 1 according to the first embodiment, and thus, the description thereof will be omitted. The drug solution administration apparatus having the liquid delivery needle tube 12A illustrated in Fig. 11 can also have the same effect as that of the drug solution administration apparatus 1 according to the above-described first embodiment.

Note that, according to the drug solution administration apparatus of the second embodiment, the number of components can be reduced compared to the drug solution administration apparatus 1 according to the first embodiment by integrally forming the connecting needle tube and the liquid delivery pipe. It is possible to reduce the weight of the drug solution administration apparatus.

Above, embodiments of the present invention, including the operation effects have been described. However, the drug solution administration apparatus of the present invention is not limited to the above-described embodiments, and various variations can be implemented within the scope of the present invention described in the claims.

In the above-described embodiments, an example has been described in which an insulin pump that administers insulin is applied as a drug solution administration apparatus, but the present invention is not limited thereto. Various other drug solutions, such as analgesics, anti-cancer drugs, HIV drugs, iron chelators, pulmonary hypertension drugs, and the like, may be used as drug solution to be administered. Not being limited to drug solution administration apparatuses that are affixed to the skin of the patient, the present invention can also be applied to other apparatuses that are used in combination with cradle devices that can be attached to the clothing or belongings of the patient. For example, body fluid component measurement apparatuses used for monitoring blood glucose levels, and the like, and patient information collection devices that collect information such as heart rate or number of steps, are exemplified, and it is needless to say that these apparatuses can be utilized in a multifaceted manner in the member communication function.

Note that, in this specification, although words such as "parallel" and "orthogonal" were used, these do not mean only strict "parallel" and "orthogonal", but may also mean a state of being "substantially parallel" or "substantially orthogonal" including "parallel" and "orthogonal" and within the range of capable of performing the functions.

### Reference Signs List

- 1: DRUG SOLUTION ADMINISTRATION APPARATUS
- 2: PUMP MAIN BODY
- 3 130 230: CRADLE DEVICE
- 6: CONNECTION PORT
- 11: HOUSING
- 12: CONNECTING NEEDLE TUBE
- 12A: LIQUID DELIVERY NEEDLE TUBE
- 13: LIQUID DELIVERY PIPE
- 14: DRUG SOLUTION STORAGE SECTION
- 15: DRIVING SECTION
- 16: POWER SUPPLY SECTION
- 17: PLUNGER MECHANISM
- 18: TRANSMISSION MECHANISM
- 21: UPPER SURFACE PORTION
- 22: BOTTOM SURFACE PORTION
- 23: FRONT SURFACE PORTION
- 24: BACK SURFACE PORTION
- 25: FIRST SIDE SURFACE PORTION
- 26: SECOND SIDE SURFACE PORTION
- 27: BOTTOM SURFACE ACCOMMODATION SECTION
- 27a: WALL SURFACE
- 28: CONNECTION OPENING
- 28a: TUBE HOLE
- 31: ENGAGEMENT PROJECTION
- 32: SLIDING GROOVE PORTION
- 33: MOUNTING DETECTION SWITCH
- 34: DETECTION GROOVE PORTION
- 35: FIRST GUIDE GROOVE PORTION
- 35a: EXTENSION SECTION
- 36: FIRST ENGAGEMENT HOOK PORTION
- 37: SECOND GUIDE GROOVE PORTION
- 38: SECOND ENGAGEMENT HOOK PORTION
- 41: PLACEMENT SURFACE PORTION
- 42: FIRST SIDE WALL PORTION
- 43: SECOND SIDE WALL PORTION
- 44: THIRD SIDE WALL PORTION
- 51: FIRST GUIDE RAIL
- 52: DETECTION RAIL
- 53: SLIDING RAIL
- 54: ENGAGEMENT HOLE
- 55: MOUNTING SECTION
- 56 57 156 157 256: POSTURE CORRECTION SECTION
- 81: PORT MAIN BODY
- 81a: CONNECTION SECTION
- 82: CAP
- 82: CANNULA
- 256b: INSERTION OPENING
- X: FIRST DIRECTION (MOUNTING DIRECTION)
- Y: SECOND DIRECTION (WIDTH DIRECTION)
- Z: THIRD DIRECTION (UP-DOWN DIRECTION)

## Claims

1. A drug solution administration apparatus (1) comprising:
a pump main body (2) having a drug solution storage section (14), a liquid delivery pipe (13) connected to the drug solution storage section (14), and a housing (11) that accommodates the drug solution storage section (14) and the liquid delivery pipe (13);
a cradle device (3, 130, 230) that has a placement surface portion (41) on which the pump main body (2) is placed, and the pump main body (2) being attachably and detachably mounted on the cradle device (3, 130, 230) from a first end toward a second end in a first direction; and
a connection port (6) that has a port main body (81) connected to the liquid delivery pipe (13) and a cannula (83) insertable into a living body, and is mounted on the placement surface portion (41), wherein
the pump main body (2) has a connecting needle tube (12) connected to the liquid delivery pipe (13) and punctured into the connection port (6),
the connection port (6) is mounted on a second end portion of the cradle device (3, 130, 230) in the first direction (X), and a connection section (81a) where the connecting needle tube (12) is punctured is oriented toward the first end in the first direction (X),
one of the housing (11) and the cradle device (3, 130, 230) is provided with a projecting guide rail formed parallel to the first direction (X),
the remaining other one of the housing (11) and the cradle device (3, 130, 230) is provided with a guide groove portion that is slidably engaged with the guide rail, and
a second end in the first direction (X) in the guide rail or the guide groove portion which is formed in the housing (11) is positioned on a second end side in the first direction (X) from a distal end of the connecting needle tube (12)
the housing (11) includes
a bottom surface portion (22) placed on the placement surface portion (41), and
a side surface portion that rises from one end portion of the bottom surface portion (22) in a second direction (Y) orthogonal to the first direction (X), in a third direction (Z) orthogonal to the first direction (X) and the second direction (Y),
the cradle device (3, 130, 230) has a side wall portion that rises from one end portion in the second direction (Y) in the placement surface portion (41) in the third direction (Z), and faces the side surface portion;
the guide rail is formed in one of the side surface portion and the side wall portion, and
the guide groove portion is formed in the remaining other one of the side surface portion and the side wall portion;
a bottom surface accommodation section (27) that accommodates the connection port (6) is formed in the bottom surface portion (22) of the housing (11);
the connecting needle tube (12) protrudes in the first direction (X) from a wall surface (27a) formed on a first end side of the bottom surface accommodation section (27);
the bottom surface accommodation section (27) is formed at a second end portion in the first direction (X) in the bottom surface portion (22) and the side surface portion,
the side surface portion has an extension section (35a) that protrudes from the wall surface (27a) of the bottom surface accommodation section (27) toward the second end in the first direction (X), and
a second end portion in the first direction (X) of the guide rail or the guide groove portion is formed in the extension section (35a); and
**characterized in that**
a protrusion length of the extension section (35a) from the wall surface (27a) is longer than a length at which the connecting needle tube (12) protrudes from the wall surface (27a),
and the length at which the guide groove portion (35) protrudes from the wall surface of housing (11) is longer than the length at which the connecting needle tube (12) protrudes from the wall surface of the housing (11),
a first posture correction section (56) is provided which is a plate-like protruding portion extending from a first side wall portion (42) and the first posture correction section (56) extends further upward in the third direction (Z) from the first side wall portion (42) and the first posture correction section (56) is formed on the first end portion side of the first guide rail (51) in a first direction (X), in the first side wall portion (42), and
a second side wall portion (43) has a second guide rail, a second posture correction section (57), and a second engagement receiving section (62), wherein the second engagement receiving section (62) is formed at a first end portion of the second side wall portion (43) in the first direction (X), wherein the second engagement receiving section (62) is an opening portion with the second side wall portion (43) cut out in a substantially rectangular shape,
the second posture correction section (57) is a plate-like protruding portion extending from the second side wall portion (43) and the second posture correction section (57) extends further upward in the third direction (Z) from the second side wall portion (43), wherein the second posture correction section (57) is formed on the first end portion side of the second guide rail in the first direction (X), in the second side wall portion (43) and the second posture correction section (57) is formed at a position facing the first posture correction section (56) formed in the first side wall portion (42),
the second posture correction section (57) protrudes in the third direction (Z) from one end portion of the second side wall portion (43) in the third direction (Z) and is curved toward one end portion in the second direction (Y).

2. The drug solution administration apparatus (1) according to claim 1, wherein
the second end in the first direction (X) in the guide rail or the guide groove portion which is formed in the cradle device (3, 130, 230) is positioned on the second end side in the first direction (X) from the connection section (81a) of the connection port (6) mounted on the cradle device (3, 130, 230).

3. The drug solution administration apparatus (1) according to claim 1 or 2, wherein
a length at which the guide rail and the guide groove portion are engaged with each other is set to be longer than a length at which the connecting needle tube (12) is punctured into the connection section (81a).

4. The drug solution administration apparatus (1) according to claim 1, wherein
a tubular connection opening (28) that protrudes in the first direction (X) is formed on the wall surface (27a) of the bottom surface accommodation section (27),
the connecting needle tube (12) is disposed in a tube hole (28a) of the connection opening (28), and
the connection section (81a) of the connection port (6) is inserted into the tube hole (28a) of the connection opening (28).

5. The drug solution administration apparatus (1) according to any one of claims 1 to 4, wherein
the connecting needle tube (12) is formed integrally with the liquid delivery pipe (13).

6. The drug solution administration apparatus (1) according to any one of claims 1 to 5 wherein
the housing (11) and the cradle device (3, 130, 230) are connected to each other attachably and detachably via a plurality of engagement structure sections, and
the engagement structure sections are formed on a first end portion side in the first direction (X) in the pump main body (2) and the cradle device (3, 130, 230).

## Patentansprüche

1. Vorrichtung (1) zur Verabreichung einer Arzneimittellösung, umfassend:
einen Pumpenhauptkörper (2), der einen Arzneimittellösungs-Speicherabschnitt (14), ein mit dem Arzneimittellösungs-Speicherabschnitt (14) verbundenes Flüssigkeitsabgaberohr (13) und ein Gehäuse (11) aufweist, das den Arzneimittellösungs-Speicherabschnitt (14) und das Flüssigkeitsabgaberohr (13) aufnimmt;
eine Umhüllungsvorrichtung (3, 130, 230), die einen Platzierungsflächenabschnitt (41) aufweist, auf dem der Pumpenhauptkörper (2) platziert wird, und der Pumpenhauptkörper (2) anbringbar und abnehmbar an der Umhüllungsvorrichtung (3, 130, 230) von einem ersten Ende in Richtung eines zweiten Endes in einer ersten Richtung angebracht ist; und
einen Verbindungsanschluss (6), der einen Anschlusshauptkörper (81), welcher mit dem Flüssigkeitsabgaberohr (13) verbunden ist, und eine Kanüle (83) aufweist, die in einen lebenden Körper einführbar ist, und der an dem Platzierungsflächenabschnitt (41) angebracht ist, wobei
der Pumpenhauptkörper (2) ein Verbindungsnadelrohr (12) aufweist, welches mit dem Flüssigkeitsabgaberohr (13) verbunden ist und in den Verbindungsanschluss (6) eingestochen wird,
der Verbindungsanschluss (6) an einem zweiten Endabschnitt der Umhüllungsvorrichtung (3, 130, 230) in der ersten Richtung (X) angebracht ist, und ein Verbindungsabschnitt (81a), an dem das Verbindungsnadelrohr (12) durchgestochen wird, in der ersten Richtung (X) zu dem ersten Ende hin ausgerichtet ist,
eines von dem Gehäuse (11) und der Umhüllungsvorrichtung (3, 130, 230) mit einer vorstehenden Führungsschiene versehen ist, die parallel zu der ersten Richtung (X) ausgebildet ist,
das verbleibende andere Teil von dem Gehäuse (11) und der Umhüllungsvorrichtung (3, 130, 230) mit einem Führungsrillenabschnitt versehen ist, der gleitend mit der Führungsschiene in Eingriff ist, und
ein zweites Ende in der ersten Richtung (X) in der Führungsschiene oder dem Führungsrillenabschnitt, der in dem Gehäuse (11) ausgebildet ist, auf einer zweiten Endseite in der ersten Richtung (X) von einem distalen Ende des Verbindungsnadelrohrs (12) positioniert ist,
wobei das Gehäuse (11) umfasst
einen Bodenflächenabschnitt (22), der auf dem Platzierungsflächenabschnitt (41) angeordnet ist, und
einen Seitenflächenabschnitt, der sich von einem Endabschnitt des Bodenflächenabschnitts (22) in einer zweiten Richtung (Y) orthogonal zu der ersten Richtung (X) in einer dritten Richtung (Z) orthogonal zu der ersten Richtung (X) und der zweiten Richtung (Y) erhebt,
die Umhüllungsvorrichtung (3, 130, 230) einen Seitenwandabschnitt aufweist, der sich von einem Endabschnitt in der zweiten Richtung (Y) in den Platzierungsflächenabschnitt (41) in der dritten Richtung (Z) erhebt und dem Seitenflächenabschnitt gegenüberliegt;
die Führungsschiene entweder in dem Seitenflächenabschnitt oder in dem Seitenwandabschnitt ausgebildet ist, und
der Führungsrillenabschnitt in dem verbleibenden anderen von dem Seitenflächenabschnitt und dem Seitenwandabschnitt ausgebildet ist;
ein Bodenflächenaufnahmeabschnitt (27), der den Verbindungsanschluss (6) aufnimmt, in dem Bodenflächenabschnitt (22) des Gehäuses (11) ausgebildet ist;
das Verbindungsnadelrohr (12) in der ersten Richtung (X) von einer Wandfläche (27a) vorsteht, die an einer ersten Endseite des Bodenflächenaufnahmeabschnitts (27) ausgebildet ist;
der Bodenflächenaufnahmeabschnitt (27) an einem zweiten Endabschnitt in der ersten Richtung (X) in dem Bodenflächenabschnitt (22) und dem Seitenflächenabschnitt ausgebildet ist,
der Seitenflächenabschnitt einen Verlängerungsabschnitt (35a) aufweist, der von der Wandfläche (27a) des Bodenflächenaufnahmeabschnitts (27) in Richtung des zweiten Endes in der ersten Richtung (X) vorsteht, und
ein zweiter Endabschnitt in der ersten Richtung (X) der Führungsschiene oder des Führungsrillenabschnitts in dem Verlängerungsabschnitt (35a) ausgebildet ist; und
**dadurch gekennzeichnet, dass**
eine Vorsprungslänge des Verlängerungsabschnitts (35a) von der Wandfläche (27a) länger ist als eine Länge, mit der das Verbindungsnadelrohr (12) von der Wandfläche (27a) vorspringt,
und die Länge, mit welcher der Führungsrillenabschnitt (35) aus der Wandfläche des Gehäuses (11) vorspringt, länger ist als die Länge, mit der das Verbindungsnadelrohr (12) aus der Wandfläche des Gehäuses (11) herausragt,
ein erster Positionierungskorrekturabschnitt (56) vorgesehen ist, der ein plattenförmig vorstehender Abschnitt ist, der sich von einem ersten Seitenwandabschnitt (42) erstreckt, und der erste Positionierungskorrekturabschnitt (56) sich weiter nach oben in der dritten Richtung (Z) von dem ersten Seitenwandabschnitt (42) erstreckt, und der erste Positionierungskorrekturabschnitt (56) an der ersten Endabschnittsseite der ersten Führungsschiene (51) in einer ersten Richtung (X) in dem ersten Seitenwandabschnitt (42) ausgebildet ist, und
ein zweiter Seitenwandabschnitt (43) eine zweite Führungsschiene, einen zweiten Positionierungskorrekturabschnitt (57) und einen zweiten Eingriffsaufnahmeabschnitt (62) aufweist, wobei der zweite Eingriffsaufnahmeabschnitt (62) an einem ersten Endabschnitt des zweiten Seitenwandabschnitts (43) in der ersten Richtung (X) ausgebildet ist, wobei der zweite Eingriffsaufnahmeabschnitt (62) ein Öffnungsabschnitt ist, wobei der zweite Seitenwandabschnitt (43) in einer im Wesentlichen rechteckigen Form ausgeschnitten ist,
der zweite Positionierungskorrekturabschnitt (57) ein plattenförmiger vorstehender Abschnitt ist, der sich von dem zweiten Seitenwandabschnitt (43) erstreckt, und der zweite Positionierungskorrekturabschnitt (57) sich weiter nach oben in der dritten Richtung (Z) von dem zweiten Seitenwandabschnitt (43) erstreckt, wobei der zweite Positionierungskorrekturabschnitt (57) an der Seite des ersten Endabschnitts der zweiten Führungsschiene in der ersten Richtung (X) in dem zweiten Seitenwandabschnitt (43) ausgebildet ist und der zweite Positionierungskorrekturabschnitt (57) an einer Position ausgebildet ist, die dem ersten Positionierungskorrekturabschnitt (56) gegenüberliegt, der in dem ersten Seitenwandabschnitt (42) ausgebildet ist,
der zweite Positionierungskorrekturabschnitt (57) in der dritten Richtung (Z) von einem Endabschnitt des zweiten Seitenwandabschnitts (43) in der dritten Richtung (Z) vorsteht und in Richtung eines Endabschnitts in der zweiten Richtung (Y) gekrümmt ist.

2. Vorrichtung (1) zur Verabreichung einer Arzneimittellösung nach Anspruch 1, wobei
das zweites Ende in der ersten Richtung (X) in der Führungsschiene oder dem Führungsrillenabschnitt, der in der Umhüllungsvorrichtung (3, 130, 230) ausgebildet ist, ist auf der zweiten Endseite in der ersten Richtung (X) von dem Verbindungsabschnitt (81a) des Verbindungsanschlusses (6) positioniert, der an der Umhüllungsvorrichtung (3, 130, 230) angebracht ist.

3. Vorrichtung (1) zur Verabreichung einer Arzneimittellösung nach Anspruch 1 oder 2, wobei
eine Länge, bei der die Führungsschiene und der Führungsrillenabschnitt miteinander in Eingriff stehen, länger eingestellt ist als eine Länge, bei der das Verbindungsnadelrohr (12) in den Verbindungsabschnitt (81a) eingestochen ist.

4. Vorrichtung (1) zur Verabreichung einer Arzneimittellösung nach Anspruch 1, wobei
eine rohrförmige Verbindungsöffnung (28), die in die erste Richtung (X) vorspringt, an der Wandfläche (27a) des Bodenflächen-Aufnahmeabschnitts (27) , ausgebildet ist,
das Verbindungsnadelrohr (12) in einem Rohrloch (28a) der Verbindungsöffnung (28) angeordnet ist, und
der Verbindungsabschnitt (81a) der Verbindungsöffnung (6) in das Rohrloch (28a) der Verbindungsöffnung (28) eingeführt ist.

5. Vorrichtung (1) zur Verabreichung einer Arzneimittellösung nach einem der Ansprüche 1 bis 4, wobei
das Verbindungsnadelrohr (12) einstückig mit dem Flüssigkeitsabgaberohr (13) ausgebildet ist.

6. Vorrichtung (1) zur Verabreichung einer Arzneimittellösung nach einem der Ansprüche 1 bis 5, wobei
das Gehäuse (11) und die Umhüllungsvorrichtung (3, 130, 230) über eine Vielzahl von Eingriffsstrukturabschnitten anbringbar und abnehmbar miteinander verbunden sind, und
die Eingriffsstrukturabschnitte an einer ersten Endabschnittseite in der ersten Richtung (X) in dem Pumpenhauptkörper (2) und der Umhüllungsvorrichtung (3, 130, 230) ausgebildet sind.

## Revendications

1. Appareil d'administration de solution médicamenteuse (1) comprenant :
un corps principal de pompe (2) comportant une section de stockage de solution médicamenteuse (14), un tuyau de distribution de liquide (13) relié à la section de stockage de solution médicamenteuse (14), et un boîtier (11) qui loge la section de stockage de solution médicamenteuse (14) et le tuyau de distribution de liquide (13) ;
un dispositif socle (3, 130, 230) comportant une partie de surface de placement (41) sur laquelle le corps principal de pompe (2) est placé, et le corps principal de pompe (2) étant monté de manière attachable et détachable sur le dispositif socle (3, 130, 230) depuis une première extrémité vers une seconde extrémité dans une première direction ; et
un orifice de liaison (6) qui comporte un corps principal d'orifice (81) relié au tuyau de distribution de liquide (13) et une canule (83) insérable dans un corps vivant, et est monté sur la partie de surface de placement (41), dans lequel
le corps principal de pompe (2) comporte un tube d'aiguille de liaison (12) relié au tuyau de distribution de liquide (13) et perforé dans l'orifice de liaison (6),
l'orifice de liaison (6) est monté sur une seconde partie d'extrémité du dispositif socle (3, 130, 230) dans la première direction (X), et une section de liaison (81a) où le tube d'aiguille de liaison (12) est perforé est orientée vers la première extrémité dans la première direction (X), l'un parmi le boîtier (11) et le dispositif socle (3, 130, 230) est pourvu d'un rail de guidage saillant formé parallèlement à la première direction (X),
l'autre parmi le boîtier (11) et le dispositif socle (3, 130, 230) est pourvu d'une partie de rainure de guidage qui est engagée en coulissant avec le rail de guidage, et une seconde extrémité dans la première direction (X) dans le rail de guidage ou dans la partie de rainure de guidage qui est formée dans le boîtier (11) est positionnée sur un second côté d'extrémité dans la première direction (X) depuis une extrémité distale du tube d'aiguille de liaison (12),
le boîtier (11) comprend
une partie de surface inférieure (22) placée sur la partie de surface de placement (41), et
une partie de surface latérale qui monte depuis une partie d'extrémité de la partie de surface inférieure (22) dans une deuxième direction (Y) orthogonale à la première direction (X), dans une troisième direction (Z) orthogonale à la première direction (X) et à la deuxième direction (Y),
le dispositif socle (3, 130, 230) comporte une partie de paroi latérale qui monte depuis une partie d'extrémité dans la deuxième direction (Y) dans la partie de surface de placement (41) dans la troisième direction (Z), et fait face à la partie de surface latérale ;
le rail de guidage est formé dans l'une parmi la partie de surface latérale et la partie de paroi latérale, et
la partie de rainure de guidage est formée dans l'autre parmi la partie de surface latérale et la partie de paroi latérale ;
une section de logement de surface inférieure (27) qui loge l'orifice de liaison (6) est formée dans la partie de surface inférieure (22) du boîtier (11) ;
le tube d'aiguille de liaison (12) fait saillie dans la première direction (X) depuis une surface de paroi (27a) formée sur un premier côté d'extrémité de la section de logement de surface inférieure (27) ;
la section de logement de surface inférieure (27) est formée au niveau d'une seconde partie d'extrémité dans la première direction (X) dans la partie de surface inférieure (22) et la partie de surface latérale,
la partie de surface latérale comporte une section d'extension (35a) qui fait saillie depuis la surface de paroi (27a) de la section de logement de surface inférieure (27) vers la seconde extrémité dans la première direction (X), et
une seconde partie d'extrémité dans la première direction (X) du rail de guidage ou de la partie de rainure de guidage est formée dans la section d'extension (35a) ; et **caractérisé en ce que**
une longueur de saillie de la section d'extension (35a) depuis la surface de paroi (27a) est plus longue qu'une longueur de laquelle le tube d'aiguille de liaison (12) fait saillie depuis la surface de paroi (27a),
et la longueur de laquelle la partie de rainure de guidage (35) fait saillie depuis la surface de paroi du boîtier (11) est plus longue que la longueur de laquelle le tube d'aiguille de liaison (12) fait saillie depuis la surface de paroi du boîtier (11),
une première section de correction de posture (56) est prévue et est une partie saillante en forme de plaque s'étendant depuis une première partie de paroi latérale (42) et la première section de correction de posture (56) s'étend davantage vers le haut dans la troisième direction (Z) depuis la première partie de paroi latérale (42) et la première section de correction de posture (56) est formée sur le premier côté de partie d'extrémité du premier rail de guidage (51) dans une première direction (X), dans la première partie de paroi latérale (42), et
une seconde partie de paroi latérale (43) comporte un second rail de guidage, une seconde section de correction de posture (57) et une seconde section de réception d'engagement (62), dans lequel la seconde section de réception d'engagement (62) est formée au niveau d'une première partie d'extrémité de la seconde partie de paroi latérale (43) dans la première direction (X), dans lequel la seconde section de réception d'engagement (62) est une partie d'ouverture avec la seconde partie de paroi latérale (43) découpée dans une forme sensiblement rectangulaire,
la seconde section de correction de posture (57) est une partie saillante en forme de plaque s'étendant depuis la seconde partie de paroi latérale (43) et la seconde section de correction de posture (57) s'étend davantage vers le haut dans la troisième direction (Z) depuis la seconde partie de paroi latérale (43), dans lequel la seconde section de correction de posture (57) est formée sur le premier côté de partie d'extrémité du second rail de guidage dans la première direction (X), dans la seconde partie de paroi latérale (43) et la seconde section de correction de posture (57) est formée au niveau d'une position faisant face à la première section de correction de posture (56) formée dans la première partie de paroi latérale (42),
la seconde section de correction de posture (57) fait saillie dans la troisième direction (Z) depuis une partie d'extrémité de la seconde partie de paroi latérale (43) dans la troisième direction (Z) et est incurvée vers une partie d'extrémité dans la seconde direction (Y).

2. Appareil d'administration de solution médicamenteuse (1) selon la revendication 1, dans lequel
la seconde extrémité dans la première direction (X) dans le rail de guidage ou dans la partie de rainure de guidage qui est formée dans le dispositif socle (3, 130, 230) est positionnée sur le second côté d'extrémité dans la première direction (X) depuis la section de liaison (81a) de l'orifice de liaison (6) monté sur le dispositif socle (3, 130, 230).

3. Appareil d'administration de solution médicamenteuse (1) selon la revendication 1 ou 2, dans lequel
une longueur de laquelle le rail de guidage et la partie de rainure de guidage sont engagés l'un avec l'autre est définie pour être plus longue qu'une longueur de laquelle le tube d'aiguille de liaison (12) est perforé dans la section de liaison (81a).

4. Appareil d'administration de solution médicamenteuse (1) selon la revendication 1, dans lequel
une ouverture de liaison tubulaire (28) faisant saillie dans la première direction (X) est formée sur la surface de paroi (27a) de la section de logement de surface inférieure (27),
le tube d'aiguille de liaison (12) est disposé dans un trou de tube (28a) de l'ouverture de liaison (28), et
la section de liaison (81a) de l'orifice de liaison (6) est insérée dans le trou de tube (28a) de l'ouverture de liaison (28).

5. Appareil d'administration de solution médicamenteuse (1) selon l'une quelconque des revendications 1 à 4, dans lequel
le tube d'aiguille de liaison (12) est formé d'un seul tenant avec le tuyau de distribution de liquide (13).

6. Appareil d'administration de solution médicamenteuse (1) selon l'une quelconque des revendications 1 à 5, dans lequel
le boîtier (11) et le dispositif socle (3, 130, 230) sont reliés l'un à l'autre de manière attachable et détachable via une pluralité de sections de structure d'engagement, et
les sections de structure d'engagement sont formées sur un premier côté de partie d'extrémité dans la première direction (X) dans le corps principal de pompe (2) et le dispositif socle (3, 130, 230).
